# EUROPEAN PATENT SPECIFICATION

(11) **EP 0 414 044 B1**
(45) Date of publication and mention of the grant of the patent: **16.03.1994**
(21) Application number: 90115189.4
(22) Date of filing: 07.08.1990
(51) Int. Cl.: C12N 15/87, C12M 1/00, C12M 3/00, A61K 47/48

(54) **Cell treating injection device**
Injektionssystem für die Behandlung von Zellen
Dispositif d'injection dans des cellules à traiter

(30) Priority: 16.08.1989 JP 211071/89
(43) Date of publication of application: 27.02.1991
(73) Proprietor: NIPPON ZEON CO., LTD., Tokyo (JP)
(72) Inventor: Igari, Minoru, Arakawa-ku, Tokyo (JP)
(74) Representative: Müller-Boré & Partner Patentanwälte

(56) References cited:
- PARTICULATE SCIENCE AND TECHNOLOGY vol. 5, no. 1, 1987, pages 27 - 37; SANFORD,J.C. et al.: "Delivery of substances into cells and tissues using a particlebombardment process"

## Description

### BACKGROUND OF THE INVENTION

The present invention relates to a cell treating injection device for injecting a cell treating material so as to carry out cell treatment such as transformation.

Such an injection device is utilized for injecting a cell treating material such as a metal fine powder having a genetic substance attached thereto at a high speed and bringing the cell treating material into collision with a sample containing a cell to be treated, so as to inject the genetic substance into the cell or generate cell fusion.

A known injection device of this kind is constructed by modifying a rivet gun. However, since the conventional injection device is a gun in itself, a propelling speed of a piston for forcing the cell treating material is too high, causing ready breakage of the device as a whole. Furthermore as the propelling speed of the piston cannot be adjusted, it is hard to handle.

### SUMMARY OF THE INVENTION

It is accordingly an object of the present invention to provide a cell treating injection device which can sufficiently reduce the propelling speed of the piston and thereby prevent the breakage of the device even in the case of using a commercially available cartridge for a rivet gun.

It is another object of the present invention to provide a cell treating injection device which can adjust the propelling speed of the piston.

According to the present invention, there is provided a cell treating injection device comprising a cylinder adapted to receive an explosive at a rear end thereof, said cylinder having a radial vent hole; an igniting mechanism for igniting said explosive; a piston adapted to be loaded into said cylinder and be propelled in said cylinder by a pressure of a combustion gas of said explosive, said piston having a cell treating material of a metal fine powder or the like attached to a front end thereof; all injection member formed from a plate body having a central small hole for injecting said cell treating material separated from said piston by collision of said piston with said injection member to a sample to be treated, said injection member being located at a front end of said cylinder and opposed to said sample with an evacuated space defined therebetween; and a variable pressure control valve communicated with said radial vent hole of said cylinder for discharging said combustion gas of said explosive according to an arbitrarily set pressure and adjusting a propelling speed of said piston.

As the above device includes the vent hole and the variable pressure valve, the combustion gas of the explosive can be discharged to thereby reduce the propelling speed of the piston. Further, the pressure of the combustion gas can be adjusted by the discharge of the gas to thereby adjust the propelling speed of the piston.

The variable pressure control valve in this device may be arbitrarily selected from any types. For example, a normal safety valve such as a chisel type or rupture disk type valve may be used so that a pressure may be suitably set by adjustment of the chisel or replacement of the disk. Preferably, the variable pressure control valve comprises a movable ring movably mounted on an outer circumference of said cylinder, said movable ring being formed with a gas discharging passage communicated with said radial vent hole of said cylinder; a spring for biasing said movable ring so as to normally urge the same against a contact member mounted on the outer circumference of said cylinder and allow said movable ring to be separated from said contact member according to the pressure of said combustion gas; and an adjuster ring threadedly engaged with the outer circumference of said cylinder for retaining an end of said spring on an opposite side of said movable ring and adjusting a biasing force of said spring to be applied to said movable ring by changing a position thereof.

According to this variable pressure control valve, the combustion gas discharged from the vent hole of the cylinder is allowed to enter the passage of the valve and urges the contact member against a biasing force of the spring to separate the movable ring from the contact member, with the result that the combustion gas is discharged from the passage of the valve. In this case, the biasing force of the spring may be adjusted by moving the position of the adjuster ring, thereby adjusting a separation distance of the movable ring from the contact member. Accordingly, an amount of the combustion gas to be discharged, that is, the pressure of the combustion gas in the cylinder may be adjusted to thereby adjust the propelling speed of the piston.

According to a preferred embodiment of the present invention, the cylinder is formed with a speed reducing radial vent hole at a position such that said speed reducing vent hole is behind said piston before said piston collides with said injection member for discharging said combustion gas of said explosive and reducing tlie propelling speed of said piston.

By the provision of the speed reducing radial vent hole, a more amount of the combustion gas may be discharged to thereby more reduce the propelling speed of the piston.

The piston to be used in the cell treating injection device is preferably formed with an elastic radial projection for elastically contacting an inner surface of said cylinder and stopping said piston at an arbitrary loading position in said cylinder.

The elastic radial projection may be constructed arbitrarily in such a manner that a rib-like projection is formed at a circumference of the rear end of the piston or a tongue-like projection is formed on the circumferential surface of the piston.

According to its construction, a distance between the explosive and the piston can be freely set upon loading of the piston. That is, a maximum pressure of the combustion gas of the explosive can be reduced by expanding the distance between the explosive and the piston. In this manner, the propelling speed of the piston can be adjusted also by adjusting the loading position of the piston.

Further, the piston is preferably formed at its front end with a central recess for collectively receiving said cell treating material to be attached thereto by a surface tension of water or the like.

When loading or propelling the piston in the cylinder, the piston advances along the vent hole or the speed reducing vent hole. In the conventional piston, as the front end surface of the piston is flat, the cell treating material is attached to the entire flat sur face, and there is a possibility that the cell treating material will be lost from the vent hole or the speed reducing vent hole upon loading or propelling the piston in the cylinder. To the contrary, according to the above construction of the piston of the present invention, the cell treating material is received in the central recess formed on the front end surface of the piston, thereby preventing the above defect. Further, as the cell treating material is collected at the position of the central small hole of the injection member upon injection, it may be effectively injected without waste.

Other objects and features of the invention will be more fully understood from the following detailed description and appended claims when taken with the accompanying drawings.

### BRIEF DESCRIPTION OF THE DRAWINGS

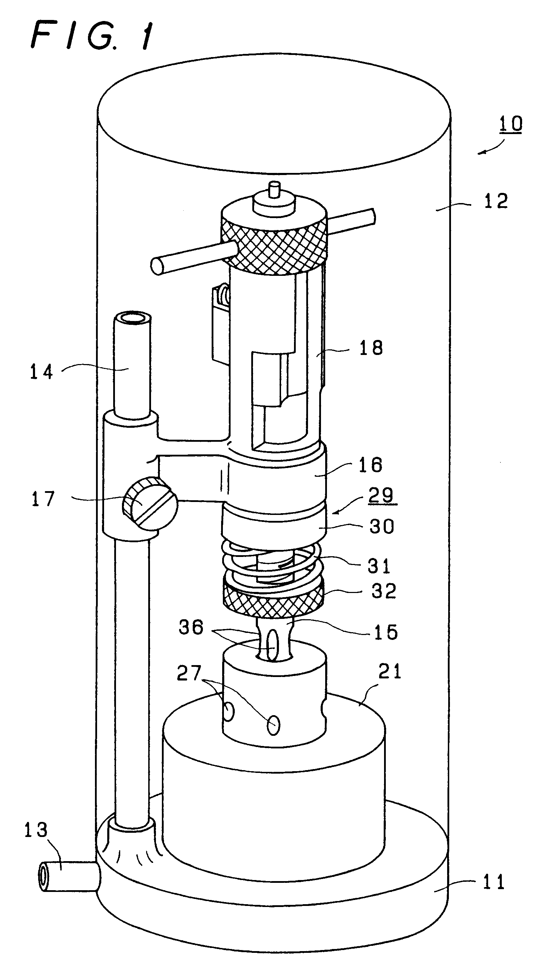

Fig. 1 is a perspective view of the cell treating injection device according to the present invention; and

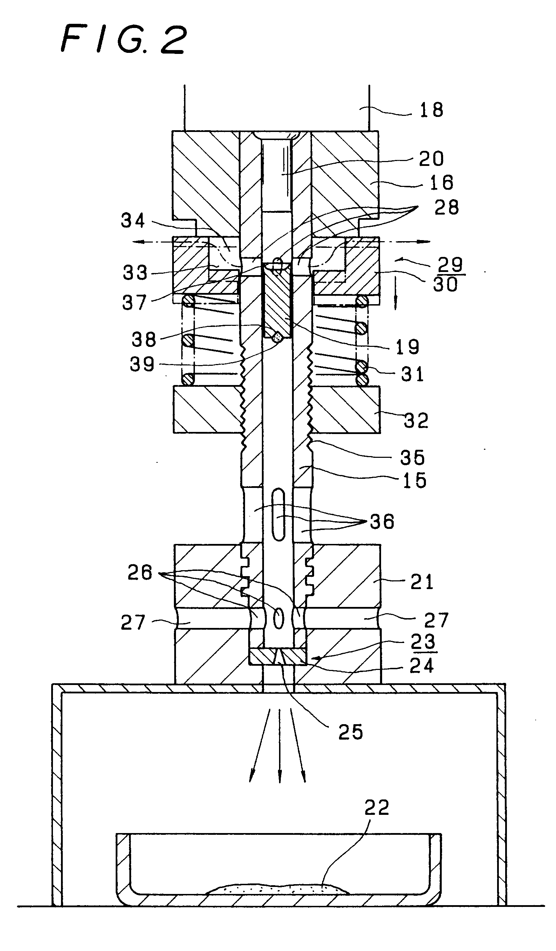

Fig. 2 is a sectional elevation of the cell treating injection device and the piston used therein according to the present invention.

### DETAILED DESCRIPTION OF THE PREFERRED EMBODIMENTS

There will now be described a preferred embodiment of the present invention with reference to the drawings.

A cell treating injection device 10 is generally covered with a detachable case 12 to be mounted on a bed 11, and the air inside the case 12 can be sucked from a suction pipe 13 by means of a vacuum pump (not shown) to obtain a vacuum condition in the case 12.

A support post 14 is provided to stand on the bed 11 aside the device 10, and a holder 16 for retaining a cylinder 15 is movably supported to the support post 14 in such a manner that a vertical position of the holder 16 may be adjusted by operating an adjusting screw 17.

Thus, the cylinder 15 vertically extends with an upper or rear portion thereof retained by the holder 16. A bolt type loading/igniting mechanism 18 is provided on the holder 16 for loading a piston 19 into the cylinder 15 and a blank cartridge 20 for loading a rivet gun behind the piston 19 and for igniting the blank cartridge 20. The ignition is carried out by a solenoid, and the operation of the mechanism 18 is carried out outside the case 12.

A cover 21 is detachably mounted to a lower or front end pottion of the cylinder 15 by means of a bayonet screw. the cover 21 is so located as to cover a sample 22 to be placed on the bed 11. An injection member 23 is fixedly sandwiched between the cover 21 and the lower end of the cylinder 15. The injection member 23 is constructed of a plate body 24 having a central tapering small hole 25. The cylinder 15 is formed at its lower end portion near the injection member 23 with a plurality of radial vent holes 26, and the cover 21 is also formed with a plurality of radial vent holes 27 communicated with the vent holes 26 of the cylinder 15.

The cylinder 15 is formed at its upper end portion with a plurality of radial vent holes 28. A variable pressure control valve 29 is mounted on the cylinder 15 so as to surround the vent holes 28. The variable pressure control valve 29 is constructed of a movable ring 30, a spring 31 and an adjuster ring 32. The movable ring 30 is vertically movably engaged with the outer circumference of the cylinder 15. An annular passage 33 is formed on an upper surface of the movable ring 30 so as to be communicated with the vent holes 28 of the cylinder 15. The spring 31 is provided between the movable ring 30 and the adjuster ring 32 to upwardly urge the movable ring 30 and make an upper end surface of the movable ring 30 in abutment against a lower end surface (contact portion) 34 of the holder 16. The adjuster ring 32 is threadedly engaged with a threaded portion 35 formed on the outer circumference of the cylinder 15 to retain a lower end of the spring 31. By manually rotating the adjuster ring 32 to adjust a vertical position thereof, a biasing force of the spring 31 to be applied to the movable ring 30 can be adjusted.

A plurality of axially-elongated radial vent holes 36 are formed at an intermediate portion of the cylinder 15 for reducing a propelling speed of the piston 19. That is, the vent holes 36 are formed at a vertical position such that they are behind the piston 19 before the piston 19 collides with the injection member 23, so that a combustion gas of explosive in the cartridge 20 after the ignition may be discharged from the vent holes 36. At this time, since the vent holes 36 are elongated in the axial direction of the cylinder 15, the combustion gas can be rapidly discharged from the vent holes 36 while the piston 19 is propelled to pass the vent holes 36.

The piston 19 is constructed of a round rod formed of synthetic resin such as nylon. The piston 19 is formed at its rear or upper peripheral edge with a rib-like elastic projection 37 projecting radially outwardly. The elastic projection 37 functions to make elastic contact of the piston 19 with the inner surface of the cylinder 15, so that the piston 19 may be stopped at a suitable position upon loading in the cylinder 15.

Further, the piston 19 is formed at its front or lower end with a central recess 38 for receiving a cell treating material 39.

Thus, the cell treating injection device 10 and the piston 19 employed therein are constructed as above, and the they are operated as follows:

First, the sample 22 containing a cell to be treated is placed on the bed 11. The holder 16 is adjusted in height to fix the injection device 10 so that the injection member 23 is located just over the sample 22 at a sui table distance. Then, the cell treating material 39, e.g. , a tungsten fine powder having a genetic substance attached thereto is attached to the recess 38 of the piston 19 by utilizing a surface tension of water. The piston 19 thus including the cell treating material 39 is loaded into the cylinder 15 by operating the loading/igniting mechanism 18. At this time, a loading position of the piston 19 is suitably set by using a rod-like jig or the like. Thereafter, the blank cartridge 20 is loaded into the cylinder 15 behind the piston 19, thus completing the loading operation. Then, the variable pressure control valve 29 is suitably adjusted in vertical position by operating the adjuster ring 32. Finally, the case 12 is placed on the bed 11 to cover the injection device 10, and the interior of the case 12 is evacuated to a reduced pressure of about 10 mmHg or lower. Thus, the preparation is completed.

Thereafter, the loading/igniting mechanism 18 is operated outside the case 12 to ignite the cartridge 20. As a result, the piston 19 is propelled by the pressure of the combustion gas of explosive in the cartridge 20 until the collision with the injection member 23. Accordingly, the cell treating material 39 attached to the piston 19 is separated from the piston 19 by the collision and is injected form the small hole 25 of the injection member 23 to collide with the sample 22, thus carrying transformation or the like of the cell in the sample 22.

In the above operation, a maximum pressure of the combustion gas can be adjusted according to the loading position of the piston 19, that is, a distance between the piston 19 and the cartridge 20 upon loading. Further, an amount of the combustion gas to be discharged from the vent holes 28 can be adjusted to the variable control valve 29. For these reasons, the propelling speed of the piston 19 can be preliminarily set to a suitable value. Moreover, the propelling speed of the piston 19 after propelling can be reduced because the combustion gas is discharged from the vent holes 36. Further, a compressed air between the piston 19 and the injection member 23 can be discharged from the vent holes 26 and 27 before the piston 19 collides with the injection member 23.

After the treatment of the sample 22, the sample 22 is taken out of the case 12, and the piston 19 is removed from the cylinder 15 by disengaging the cover 21 from the cylinder 15. The piston removed is discarded. The cartridge 20 is also removed from the cylinder 15 by operating the loading/igniting mechanism 18.

As described above, the cell treating injection device of the present invention includes the vent holes and the variable pressure control valve communicated with the vent holes. Accordingly the propelling speed of the piston can be adjusted so as to inject the cell treating material at a desired injection speed. Further, as the propelling speed can be reduced, there is no danger of breakage of the device, and a commercially available blank cartridge for a rivet gun can be used. Particularly in the case that the variable pressure control valve is constructed of the movable ring, the spring and the adjuster ring as mentioned above, the construction of the valve can be made simple, which will contribute to easy handling. Further, the propelling speed of the piston can be more reduced by providing the speed reducing vent holes.

As described above, the piston of the present invention has the elastic projection. Accordingly, the piston can be stopped at a suitable position in the cylinder upon loading. That is, the distance between the piston and the cartridge containing the explosive can be suitably set to adjust a maximum pressure of the combustion gas and thereby adjust the propelling speed of the piston. In combination with the pressure adjustment by the variable pressure control valve, the propelling speed of the piston can be greatly adjusted. Furthermore, as the central recess is formed on the front end surface of the piston, the cell treating material can be concentrated at the central portion of the front end surface of the piston. Accordingly, there is no possibility of the cell treating material being lost from the vent holes of the cylinder, and the cell treating material can be almost injected from the central small hole of the injection member.

While the invention has been described with reference to a specific embodiment, the description is illustrative and is not to be construed as limiting the scope of the invention. Various modifications and changes may occur to those skilled in the art without departing from the spirit and scope of the invention as defined by the appended claims.

## Claims

1. A cell treating injection device comprising a cylinder adapted to receive an explosive at a rear end thereof, said cylinder having a radial vent hole; an igniting mechanism for igniting said explosive; a piston adapted to be loaded into said cylinder and be propelled in said cylinder by a pressure of a combustion gas of said explosive, said piston having a cell treating material of a metal fine powder or the like attached to a front end thereof; an injection member formed from a plate body having a central small hole for injecting said cell treating material separated from said piston by collision of said piston with said injection member to a sample to be treated, said injection member being located at a front end of said cyiinder and opposed to said sample with an evacuated space defined therebetween; and a variable pressure control valve communicated with said radial vent hole of said cyiinder for discharging said combustion gas of said explosive according to an arbitrarily set pressure and adjusting a propelling speed of said piston.

2. The cell treating injection device as defined in claim 1, wherein said variable pressure control valve comprises a movable ring movably mounted on an outer circumference of said cylinder, said movable ring being formed with a gas discharging passage communicated with said radial vent hole of said cylinder; a spring for biasing said movable ring so as to normally urge the same against a contact member mounted on the outer circumference of said cylinder and allow said movable ring to be separated from said contact member according to the pressure of said combustion gas; and an adjuster ring threadedly engaged with the outer circumference of said cylinder for retaining an end of said spring on an opposite side of said movable ring and adjusting a biasing force of said spring to be applied to said movable ring by changing a position thereof.

3. The cell treating injection device as defined in claim 1 or 2, wherein said cylinder is formed with a speed reducing radial vent hole at a position such that said speed reducing vent hole is beliind said piston before said piston collides with said injection member for discharging said combustion gas of said explosive and reducing the propelling speed of said piston.

4. The cell treating injection device as defined in claim 1 or 2, wherein said piston is formed with an elastic radial projection for elastically contracting an inner surface of said cylinder and stopping said piston at an arbitrary loading position in said cylinder.

5. The cell treating injection device as defined in claim 3, wherein said piston is formed with an elastic radial projection for elastically contacting an inner surface of said cylinder and stopping said piston at an arbitrary loading position in said cylinder.

6. The cell treating injection device as defined in claim 1 or 2, wherein said piston is formed at its front end with a central recess for collectively receiving said cell treating material to be attached thereto by a surface tension of water or the like.

7. The cell treating injection device as defined in claim 3, wherein said piston is formed at its front end with a central recess for collectively receiving said cell treating material to be attached thereto by a surface tension of water or the like.

## Patentansprüche

1. Injektionsvorrichtung für die Behandlung von Zellen mit einem Zylinder zur Aufnahme eines Explosivstoffes an einem hinteren Ende von diesem, der eine radiale Lüftungsöffnung aufweist, einem Zündungsmechanismus zum Zünden des Explosivstoffes, einem Kolben, der in den Zylinder geladen und in dem Zylinder durch einen Druck eines Verbrennungsgases des Explosivstoffes angetrieben werden kann, wobei der Kolben ein Zellbehandlungsmaterial aus einem feinen Metallpulver oder dergleichen aufweist, das an einem Stirnende von diesem angebracht ist, einem Injektionsglied, das aus einem Plattenkörper gebildet wird, der eine zentrale kleine Öffnung zum Injizieren des Zellbehandlungsmaterials in eine zu behandelnde Probe aufweist, das von dem Kolben durch Kollision des Kolbens mit dem Injektionsglied getrennt wird, wobei das Injektionsglied an einem Stirnende des Zylinders und gegenüber der Probe lokalisiert ist, wobei ein dazwischen evakuierter Raum begrenzt ist, und einem Steuerventil für variablen Druck, das mit der radialen Lüftungsöffnung des Zylinders zum Ausbringen des Verbrennungsgases des Explosivstoffes in Abhängigkeit von einem willkürlich eingestellten Druck und zum Einstellen einer Antriebsgeschwindigkeit für den Kolben verbunden ist.

2. Injektionsvorrichtung nach Anspruch 1, wobei das Steuerventil für variablen Druck einen beweglichen Ring umfaßt, der auf einem äußeren Umfang des Zylinders beweglich montiert ist, wobei der bewegliche Ring mit einem Gasausbringungsdurchgang ausgebildet ist, der mit der radialen Lüftungsöffnung des Zylinders in Verbindung steht, einer Feder zum Vorspannen des beweglichen Rings, so daß derselbe normalereise gegen ein Kontaktglied gedrängt ist, das auf dem äußeren Kreisumfang des Zylinders montiert ist und dem beweglichen Ring erlaubt, von dem Kontaktglied in Abhängigkeit vom Druck des Verbrennungsgases getrennt zu werden, und einen Einstellerring, der mit dem äußeren Umfang des Zylinders im Gewindeeingriff steht, um ein Ende der Feder an einer entgegengesetzten Seite des beweglichen Rings zu halten und eine Vorspannkraft der Feder, die an den beweglichen Ring aufgelegt werden soll durch Ändern einer Position von diesem einzustellen.

3. Injektionsvorrichtung nach Anspruch 1 oder 2, wobei der Zylinder mit radialer Lüftungsöffnung zum Geschwindigkeitsreduzieren an einer Position ausgebildet ist, so daß die Lüftungsöffnung zum Geschwindigkeitsreduzieren sich hinter dem Kolben befindet, bevor der Kolben mit dem Injektionsglied kollidiert, zum Ausbringen des Verbrennungsgases des Explosivstoffes und Reduzieren der Antriebsgeschwindigkeit des Kolbens.

4. Injektionsvorrichtung nach Anspruch 1 oder 2, wobei der Kolben mit einem elastischen radialen Vorsprung zum elastischen Kontaktieren einer inneren Oberfläche des Zylinders und Anhalten des Kolbens bei einer willkürlichen Füllposition des Zylinders ausgebildet ist.

5. Injektionsvorrichtung nach Anspruch 3, wobei der Kolben mit einem elastischen radialen Vorsprung zum elastischen Kontaktieren einer inneren Oberfläche des Zylinders und Anhalten des Kolbens bei einer willkürlichen Füllposition des Zylinders ausgebildet ist.

6. Injektionsvorrichtung nach Anspruch 1 oder 2, wobei der Kolben an seiner Stirnseite mit einer zentralen Vertiefung zum kollektiven Aufnehmen des Zellbehandlungsmaterials, das daran durch eine Oberflächenspannung von Wasser oder dergleichen angebracht werden soll, ausgebildet ist.

7. Injektionsvorrichtung nach Anspruch 3, wobei der Kolben an seiner Stirnseite mit einer zentralen Vertiefung zum kollektiven Aufnehmen des Zellbehandlungsmaterials, das daran durch eine Oberflächenspannung von Wasser oder dergleichen angebracht werden soll, ausgebildet ist.

## Revendications

1. Dispositif d'injection dans des cellules à traiter comprenant un cylindre adapté à recevoir un explosif à son extrémité arrière, ledit cylindre comportant un évent radial; un mécanisme d'allumage pour mettre feu audit explosif; un piston adapté à être chargé dans ledit cylindre et à être propulsé dans ledit cylindre par la pression d'un gaz de combustion dudit explosif, ledit piston comportant une matière de traitement de cellules d'une fine poudre de métal ou analogue fixée à son extrémité avant; un organe d'injection composé d'un corps de plaque comportant un petit orifice central pour injecter ladite matière de traitement de cellules, séparé dudit piston, par collision dudit piston avec ledit organe d'injection dans un échantillon à traiter, ledit organe d'injection étant situé à une extrémité avant dudit cylindre et en face dudit échantillon avec un espace sous vide formé entre eux; et une valve de régulation de pression variable communique avec ledit évent radial dudit cylindre pour distribuer ledit gaz de combustion dudit explosif en fonction d'une pression fixée arbitrairement et ajuster la vitesse de propulsion dudit piston.

2. Dispositif d'injection dans des cellules à traiter selon la revendication 1, dans lequel ladite valve de régulation de pression variable comprend une bague mobile montée de manière à être amovible sur une circonférence externe dudit cylindre, ladite bague mobile étant dotée d'un passage de distribution de gaz en communication avec ledit évent radial dudit cylindre; un ressort pour solliciter ladite bague mobile afin d'amener normalement celle-ci contre un organe de contact monté sur la circonférence externe dudit cylindre et de permettre à ladite bague mobile d'être séparée dudit organe de contact selon la pression dudit gaz de combustion; et une bague de réglage engagée par vissage avec la circonférence externe dudit cylindre pour retenir une extrémité dudit ressort sur une face opposée de ladite bague mobile et ajuster une force de sollicitation dudit ressort devant être appliquée sur ladite bague mobile en changeant la position de celle-ci.

3. Dispositif d'injection dans des cellules à traiter selon la revendication 1 ou 2, dans lequel ledit cylindre est doté d'un évent radial de réduction de vitesse en une position de manière que ledit évent de réduction de vitesse se situe derrière ledit piston avant que ledit piston entre en collision avec ledit organe d'injection pour distribuer ledit gaz de combustion dudit explosif et réduire la vitesse de propulsion dudit piston.

4. Dispositif d'injection dans des cellules à traiter selon la revendication 1 ou 2, dans lequel ledit piston est doté d'une projection radiale élastique pour contacter élastiquement une surface interne dudit cylindre et stopper ledit piston à une position de chargement arbitraire dans ledit cylindre.

5. Dispositif d'injection dans des cellules à traiter selon la revendication 3, dans lequel ledit piston est doté d'une projection radiale élastique pour contacter élastiquement une surface interne dudit cylindre et stopper ledit piston à une position de chargement arbitraire dans ledit cylindre.

6. Dispositif d'injection dans des cellules à traiter selon la revendication 1 ou 2, dans lequel ledit piston est doté à son extrémité avant d'un évidement central pour concentrer ladite matière de traitement de cellules à fixer à celui-ci par une tension superficielle d'eau ou analogue.

7. Dispositif d'injection dans des cellules à traiter selon la revendication 3, dans lequel ledit piston est doté à son extrémité avant d'un évidement central pour concentrer ladite matière de traitement de cellules à fixer à celui-ci par une tension superficielle d'eau ou analogue.
